# EUROPÄISCHE PATENTSCHRIFT

(11) **EP 0 668 265 B1**
(45) Veröffentlichungstag und Bekanntmachung des Hinweises auf die Patenterteilung: **18.06.1997**
(21) Anmeldenummer: 95101403.4
(22) Anmeldetag: 02.02.1995
(51) Int. Cl.: C07C 323/52, C07C 323/12, C07C 323/66, C07C 323/59, C04B 41/51, C03C 17/10

(54) **Monoedelmetall-dithiolate und deren Verwendung zum Herstellen edelmetallhaltiger Dekore auf einbrennfähigen Unterlagen**
Mono-noble-metal dithiolates and their use in the preparation of noble metal containing decorations on substrates capable of being fired
Dithiolates de monometal noble et leur utilisation pour la préparation des décorations contenant des métaux nobles sur des substrats aptes à la cuisson

(30) Priorität: 21.02.1994 DE 4405423; 24.02.1994 DE 4405933
(43) Veröffentlichungstag der Anmeldung: 23.08.1995
(73) Patentinhaber: CERDEC AKTIENGESELLSCHAFT KERAMISCHE FARBEN, D-60327 Frankfurt (DE)
(72) Erfinder: Schulz, Andreas, Dr., D-63263 Neu-Isenburg (DE); Höfler, Marco, D-63579 Freigericht (DE)

(56) Entgegenhaltungen:
- EP-A- 0 491 143
- EP-A- 0 514 073
- JOURNAL OF THE CHEMICAL SOCIETY, CHEMICAL COMMUNICATIONS, Nr. 24, 15.Dezember 1976 LETCHWORTH, GB, Seiten 1051-1052, A.A. ISAB, ET AL.: '13C Nuclear magnetic resonance detection of thiol exchange on gold(I): significance in chemotherapy'
- CHEMICAL ABSTRACTS, vol. 51, no. 7, 10.April 1957 Columbus, Ohio, US; abstract no. 5692h, V.E. PETRUN'KIN: 'Synthesis and properties of dimercapto derivatives of alkanesulphonic acids. I. Synthesis of sodium 2,3-dimercaptopropanesulphonate (Unithiol) and sodium mercaptoethane- sulphonate' & UKRAINS'KII KHIMICHNII ZHURNAL, 1956, 22, 603-607

## Beschreibung

Die Erfindung richtet sich auf Monoedelmetall-dithiolate, insbesondere wasserlösliche Monogold(I)-dithiolate, deren Herstellung und Verwendung zum Herstellen edelmetallhaltiger Dekore, insbesondere hochglänzender Dekore aus einem zusammenhängenden Edelmetallfilm, auf einbrennfähigen Unterlagen. Weitere Gegenstände der Erfindung betreffen edelmetallhaltige Präparate sowie Abziehbilder, welche jeweils mindestens ein erfindungsgemäßes Monoedelmetall-dithiolat enthalten, zum Dekorieren einbrennfähiger Unterlagen.

Zur Herstellung eines Edelmetalldekors, insbesondere eines im wesentlichen Gold und/oder Silber in Form eines hochglänzenden Metallfilms ausgebildeten Dekors, worunter auch Leiterbahnen aus insbesondere Gold in integrierten Schaltkreisen zählen, auf einem einbrennfähigen Substrat finden seit langem sogenannte Edelmetallpräparate, beispielsweise Glanzgoldpräparate und Poliergoldpräparate, Anwendung. Solche Präparate enthalten im allgemeinen eine oder mehrere, in einem rein organischen oder organischwäßrigen Trägermedium lösliche schwefelorganische Edelmetallverbindungen, sogenannte Edelmetall-thiolate, wobei das Edelmetall im Falle von Ag und Au in der einwertigen Oxidationsstufe vorliegt. Die genannten Präparate enthalten außer der Edelmetallverbindung, dem Lösungsmittel und einem oder mehreren organischen polymeren Bindemitteln zusätzlich ein oder mehrere Flußmittel, wie beispielsweise sogenannte Resinate und/oder einfache Salze, Oxide oder Koordinationsverbindungen eines oder mehrerer der Elemente Bor, Silicium, Vanadium, Chrom, Indium, Zink, Antimon, Wismut und Rhodium sowie Hilfsstoffe zur Einstellung der Verarbeitungs- und Gebrauchseigenschaften. Die Edelmetallpräparate werden mittels üblicher direkter und indirekter Druckverfahren, Sprühen oder Pinseln oder unter Verwendung der Abziehbildtechnik auf die zu beschichtende Oberfläche aufgetragen. Nach dem Abdunsten des Lösungsmittels schließt sich ein Brennvorgang bei einer auf das Substrat und das Goldpräparat abgestimmten Temperatur an - meistens liegt die maximale Brenntemperatur zwischen 400 und 900 °C, jedoch sind in Sonderfällen auch höhere Temperaturen möglich. Durch den Brennvorgang wird ein Edelmetallfilm ausgebildet und auf der Oberfläche des Substrats fixiert. Es ist der Fachwelt wohlbekannt, daß zum Erhalt hochglänzender Edelmetallfilme das verwendete Edelmetall-thiolat, das Bindemittel- und das Lösungsmittelsystem sorgfältig aufeinander abgestimmt werden müssen, um zu brillanten und auf dem Substrat festhaftenden dekorbildenden Filmen, worunter auch Leiterbahnen für elektrische/elektronische Zwecke verstanden werden, zu kommen.

Bei den schwefelorganischen Verbindungen für die genannten Dekorationspräparate, handelte es sich lange Zeit fast ausschließlich um sogenannte Goldsulforesinate, welche aus einem Goldsalz und geschwefelten, insbesondere natürlich vorkommenden Terpenen gewonnen wurden. In neuer Zeit haben synthetisch hergestellte Edelmetall-thiolate, insbesondere Gold(I)-thiolate der allgemeinen Formel Au-S-R an Bedeutung gewonnen; R steht hierin insbesondere für eine Alkyl-, Cycloalkyl-, Aryl- oder Aralkylgruppe oder einen bicyclischen Kohlenwasserstoffrest. Die genannten Gold(I)-thiolate erforderten im allgemeinen die Verwendung eines rein organischen Lösungsmittelsystems - beispielhaft wird auf die EP-B 0 491 143 verwiesen.

Aus arbeitshygienischen, Sicherheits- und Umweltgründen besteht ein wachsendes Interesse an Edelmetallpräparaten zum Dekorieren einbrennfähiger Substrate, in deren Lösungsmittelsystem zumindest ein Teil des organischen Lösungsmittels durch Wasser ersetzt ist. So ist aus der DE-OS 32 17 049 ein Anstrichmittel zum Auftragen einer Überglasurdekoration auf Porzellan bekannt, das 15 bis 40 Gew.-% Polyvinylpyrrolidon oder ein Gemisch aus Polyvinylpyrrolidon und wäßrigem Polyethylenoxid, 45 bis 85 Gew.-% Ethylenglykol und/oder Propylenglykol und eventuell Wasser enthält. Als färbende Stoffe werden in diesem Dokument Oxide, Gold und organische Goldverbindungen genannt, ohne jedoch irgendeine Struktur der Goldverbindungen anzugeben.

Ein Polyvinylpyrrolidon und Wasser enthaltendes Poliergoldpräparat, das als Bindemittel zusätzlich eine wäßrige Acrylatharz-Dispersion und als färbende Komponente Goldpulver und/oder eine schwer lösliche Goldverbindung enthält, ist aus der GB-A 22 16 536 bekannt. Ausweislich der Beispiele sind auch in diesen Präparaten stets alkoholische Lösungsmittel anwesend. Zusätzlich enthalten die beispielhaften Präparate ein nichtionogenes Netzmittel in einer Menge von 5 bzw. 6 Gew.-%. Eine Anregung, eine wasserlösliche Goldverbindung einzusetzen und auf die Anwesenheit von Glykolen und Alkoholen zu verzichten, läßt sich diesem Dokument nicht entnehmen. Edelmetallpräparate auf der Basis der vorgenannten Dokumente führen, wie die Praxis zeigte, vielfach zu Dekoren, welche einen ungenügenden Glanz aufweisen.

Aus der EP-A 0 514 073 sind homogene Zusammensetzungen, vorzugsweise Lösungen, bekannt, welche beim Einbrennen einen glänzenden metallischen Edelmetallfilm ausbilden. Die Zusammensetzungen enthalten 3 bis 22 Gew.-% eines Edelmetall-thiolats, ein polymeres Harz und als Lösungsmittelsystem ein Gemisch aus Wasser und einem organischen Lösungsmittel (Cosolvens), bei welchem es sich vorzugsweise um wassermischbare Alkohole, Ether oder Ester handelt. Sowohl das Edelmetall-thiolat als auch das Bindemittel sollen in dem Wasser/Cosolvens-Gemisch löslich sein. Bei den bevorzugt eingesetzten Gold(I)-thiolaten handelt es sich um solche der allgemeinen Formel Au-S-R-H oder Au-S-R-X, wobei X für eine Nitrogruppe oder -COOH, -SO₂OH, -OH, -CONH₂, -NH₂ oder -O-P(O)(OH)₂, wobei die H-Atome gegebenenfalls substituiert sein können, oder Salze davon und R für einen divalenten organischen Rest stehen. Aus dem genannten Dokument geht anhand zahlreicher Beispiele und Vergleichsbeispiele deutlich hervor, daß nur ganz spezielle Gold(I)-thiolate in entsprechenden Goldpräparaten zu Dekoren mit gutem Glanz und guter Haftfestigkeit auf der dekorierten Unterlage führen. Ausweislich der in diesem Dokument beschriebenen Vergleichsbeispiele konnten unter Verwendung einiger unter die vorgenannten Formeln fallender Gold(I)-thiolate, darunter beispielsweise Gold(I)-mercaptobernsteinsäure nur stumpf aussehende Dekore erhalten werden.

Aufgabe der vorliegenden Erfindung ist demgemäß, eine weitere Gruppe von Edelmetall-thiolaten aufzuzeigen, welche sich zur Herstellung hochglänzender Edelmetalldekore auf einbrennfähigen Unterlagen eignen. Die neuen Edelmetall-thiolate sollten ihre Wirkung in Glanzedelmetallpräparaten mit einem wäßrig-organischen und insbesondere rein wäßrigen Lösungsmittelsystem entfalten. Die neuen Edelmetall-thiolate sollten sicher anwendbar sein und zu ästhetisch hochwertigen, insbesondere hochglänzenden, poren- und fleckenfreien, gut haftenden Dekoren auf einbrennfähigen Substraten führen.

Es wurde überraschenderweise gefunden, daß durch die Einführung einer zweiten Sulfhydrylgruppe in die dem Edelmetall-thiolat zugrundeliegende organische Verbindung die Eignung des Edelmetall-thiolats als Bestandteil von Edelmetall-Dekorationspräparaten zur Herstellung hochglänzender Dekore wesentlich verbessert werden konnte. Gegenstand der vorliegenden Erfindung sind demgemäß Monoedelmetall-dithiolate der allgemeinen Formel (A) 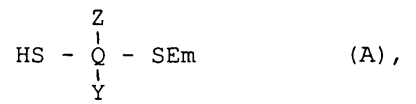 worin bedeuten
- Em:: ein Edelmetall aus der Reihe Au I oder Ag I oder ein Äquivalent von Pd II, Pt II oder Rh III
- Q:: ein tetravalenter organischer Rest mit 2 bis 10 C-Atomen
- Y:: eine hydrophile Gruppe aus der Reihe -COOH, -COO⁻Kat⁺, wobei Kat⁺ für Li⁺, Na⁺, K⁺, NH₄⁺, (C₁- bis C₃-Alkyl)ₙ N⁺H₍₄₋ₙ₎ oder (Hydroxy-(C₁- bis C₃-)alkyl)ₙ N⁺H₍₄₋ₙ₎ und n für eine ganze Zahl zwischen 1 und 4 stehen, oder 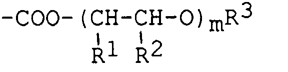 mit R¹,R² und R³ gleich oder verschieden H oder Methyl und m gleich eine ganze Zahl von 1 bis 12,
- Z:: gleich Y oder -H oder eine Gruppe aus der Reihe -OR', -SR', -SO₃R', -NR'₂, NR'₃⁺X⁻, wobei X⁻ für ein Mineralsäure- oder Carbonsäureanion und R' für Wasserstoff oder die Gruppe 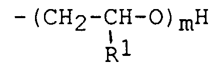 stehen und R¹ und m die vorgenannte Bedeutung haben.

Bevorzugte Monoedelmetall-dithiolate enthalten als Edelmetall Gold oder Silber, insbesondere Gold.

Der tetravalente organische Rest Q und die Substituenten Z und Y werden vorzugsweise derart ausgewählt, daß das resultierende Monoedelmetall-dithiolat eine hohe Löslichkeit in Wasser aufweist. Eine gute Wasserlöslichkeit wird insbesondere dann erhalten, wenn Y und/oder Z COO⁻Kat⁺ bedeuten.

Sofern in den erfindungsgemäßen Monoedelmetall-dithiolaten Y und/oder Z für -COO⁻Kat⁺ bedeutet, werden die genannten Ammoniumkationen gegenüber den Alkalimetallkationen bevorzugt. Im Falle der Alkylammoniumkationen kann Alkyl Methyl, Ethyl, n-Propyl oder i-Propyl bedeuten; bei den Alkylammoniumkationen kann es sich um Mono-, Di-, Tri- oder Tetra-alkylammonium mit den vorgenannten Alkylgruppen, welche auch gemischt sein können, handeln. Bei den Mono-, Di-, Tri- oder Tetra-hydroxyalkylammoniumkationen steht die Hydroxyalkylgruppe für Hydroxymethyl, Hydroxyethyl, 2-Hydroxypropyl oder 3-Hydroxypropyl. Ausgehend von Y und/oder Z gleich Carboxyl lassen sich die genannten Salze in einfacher Weise durch eine übliche Neutralisationsreaktion unter Verwendung des entsprechenden Amins oder Ammoniumhydroxids erzeugen.

Soweit Y für die Gruppe -COO(CHR¹-CHR²-O)ₘR³ steht, bedeutet m vorzugsweise eine ganze Zahl zwischen 3 und 7. Monoedelmetall-dithiolate mit Z gleich Wasserstoff weisen naturgemäß eine geringere Wasserlöslichkeit und damit eine geringere Eignung für wasserhaltige Edelmetallpräparate auf als Verbindungen, in welchen Z selbst eine hydrophile Gruppe darstellt.

Der organische Rest Q enthält zwischen 2 und 10 C-Atomen; der Rest kann zusätzlich Sauerstoff-, Stickstoff- oder Schwefelatome enthalten, beispielsweise in Form von Hydroxyl-, Carboxyl-, Alkoxy-, Amino-, Alkylamino-, Mercapto- oder Alkylmercaptogruppen. Besonders bevorzugt handelt es sich bei dem organischen Rest Q um einen C₂-, C₃- oder C₄-alkantetraylrest. Gemäß einer weiteren Ausführungsform ist der organische Rest Q ein 5- oder 6-gliedriger cycloaliphatischer Tetraylrest, wobei ein C-Ringglied durch Sauerstoff (-O-) oder eine Iminogruppe (-NH- oder -NR-) ersetzt sein kann; bei den cyclischen Resten Q handelt es sich somit um Cyclohexantetrayl, Cyclopentantetrayl, Tetrahydrofurantetrayl, Pyrrolidintetrayl, Piperidintetrayl oder Tetrahydropyrantetrayl.

In den erfindungsgemäßen Monoedelmetall-dithiolaten können die SH-Gruppe und SEm-Gruppe an benachbarten Kohlenstoffatomen oder an durch ein oder mehrere Glieder voneinander getrennten Kohlenstoffatomen gebunden sein. Verbindungen, in welchen die SH- und SEm-Gruppe an benachbarten Kohlenstoffatomen stehen, sind besonders bevorzugt. Demgemäß sind Monoedelmetall-dithiolate, insbesondere Monogold(I)- und Monosilber-dithiolate von Dimercaptobernsteinsäure, 2,3-Dimercaptoglutarsäure und 2,3- oder 3,4- oder 2,5-Dimercaptoadipinsäure sowie deren Salzen mit dem Kation Kat⁺ besonders bevorzugt. Es ist besonders zweckmäßig, ein Salz aus der Monoedelmetall-dimercaptobernsteinsäure mit einem Mono-, Di- oder Tri-(C₁- bis bis C₃-)alkylamin oder Mono-, Di- oder Tri-(hydroxy-(C₁-C₃₋)alkyl)amin zu bilden. Zur Salzbildung ist ein Amin aus der Reihe Mono-, Di- oder Triethanolamin sowie Triethyl-, Tri-n- oder Tri-iso-Propylamin in einer zur Neutralisation der anwesenden Carboxylgruppen ausreichenden Menge besonders geeignet. Die genannten Amine sind auch zur Salzbildung mit anderen erfindungsgemäßen Monoedelmetalldithiolaten geeignet.

Monoedelmetall-dithiolate mit der zuvor aufgezeigten Struktur sind bisher nie beschrieben und damit auch nicht für die Verwendung zum Herstellen metallischer edelmetallhaltiger Dekore auf einbrennfähigen Unterlagen vorgeschlagen worden. Aufgrund des Eigenschaftsvergleichs von Dekoren, welche einerseits mit erfindungsgemäßen Monoedelmetall-dithiolaten und andererseits mit vorbekannten strukturähnlichen Edelmetall-thiolaten, welche keine zusätzliche SH-Gruppe aufweisen, hergestellt wurden, wird angenommen, daß die erfindungsgemäßen Monoedelmetall-thiolate durch die zusätzliche SH-Gruppe derart stabilisiert werden, daß während des Einbrennvorgangs der auf eine einbrennfähige Unterlage, wie Glas, Porzellan und Keramik, aufgetragenen Edelmetallverbindung ein zusammenhängender, festhaftender und hochglänzender Edelmetallfilm ausgebildet werden kann.

Die Herstellung der erfindungsgemäßen Monoedelmetall-dithiolate der allgemeinen Formel (A') 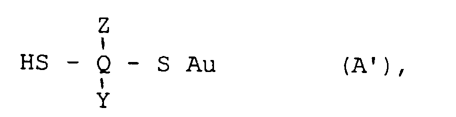 worin Q, Y und Z die vorgenannte Bedeutung haben, erfolgt in prinzipiell analoger Weise, wie dies aus der Herstellung vorbekannter Edelmetall-thiolate der Fachwelt bekannt ist - siehe die eingangs zitierten Dokumente. Im Falle der Monogold(I)-dithiolate wird aus Tetrachlorogoldsäure nach Zugabe der doppelt äquivalenten Menge eines Thioethers R''SR''' ein Gold(I)-komplex der Formel AuCl(R''SR''') gebildet; der genannte Komplex wird sodann in Gegenwart eines Lösungsmittels mit einer im wesentlichen äquivalenten Molmenge eines Dithiols (Dimercaptoverbindung) der allgemeinen Formel (B) umgesetzt 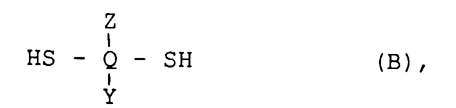 wobei Q, Y und Z wiederum die gleiche Bedeutung haben, wie in der Formel (A'). Ein besonders zweckmäßiger Thioether für die Bildung des Gold(I)-komplexes ist Methionin.

Die Herstellung der Dithiolverbindung (B) erfolgt unter Heranziehung üblicher Verfahrensstufen: Beispielsweise lassen sich Dimercaptoalkandicarbonsäuren durch Umsetzung der entsprechenden Alkindicarbonsäuren mit Thioessigsäure gewinnen; eine weitere Zugangsmöglichkeit für aliphatische und cyclische Dimercaptoverbindungen der allgemeinen Struktur (B) besteht auch darin, die entsprechende Dihalogenverbindung (Z-Q(Hal)₂-Y) mit einem Alkalisulfid umzusetzen. Erfindungsgemäße Ag-Verbindungen lassen sich in einfacher Weise durch Umsetzung eines löslichen Ag-Salzes mit einem Dithiol (B) gewinnen. In entsprechender Weise sind erfindungsgemäße Pd- und Pt-Verbindungen aus einem Pd(II)- beziehungsweise Pt(II)-Salz und dem Dithiol, hier bei einem Molverhältnis von etwa 1 zu 2, erhältlich.

Die erfindungsgemäßen Monoedelmetall-dithiolate lassen sich zweckmäßigerweise in Form edelmetallhaltiger Präparate zum Dekorieren einbrennfähiger Unterlagen, insbesondere Glas, Porzellan und Keramik, verwenden. Solche Präparate enthalten mindestens ein erfindungsgemäßes Monoedelmetall-dithiolat, ein oder mehrere polymere organische Bindemittel sowie Lösungsmittel, vorzugsweise ein wäßrig-organisches oder besonders bevorzugt ein rein wäßriges Lösungsmittelsystem, um hierin die organischen Polymeren sowie das Monoedelmetall-dithiolat zu lösen oder anderweitig eine homogene Verteilung zu gewährleisten. Soweit erforderlich, enthalten die edelmetallhaltigen Präparate zusätzlich Hilfsstoffe, um die gewünschten Verarbeitungseigenschaften, etwa eine siebdruckfähige Viskosität und eine hohe Trocknungsgeschwindigkeit des Präparats, zu erzielen und ganz bestimmte optische - Farbnuancen - und Gebrauchseigenschaften, wie Haftung auf dem Untergrund, einzustellen.

Unter Verwendung ausgewählter erfindungsgemäßer Monoedelmetall-dithiolate lassen sich im wesentlichen rein wäßrige Edelmetallpräparate erzeugen. Derartige Präparate sind dadurch gekennzeichnet, daß sie als Lösungsmittel Wasser und weniger als 2 Gew.-%, bezogen auf das Präparat, organische Lösungsmittel enthalten, das Bindemittel wasserlöslich ist, das Monoedelmetall-dithiolat mindestens eine zur Salzbildung befähigte funktionelle Gruppe aufweist und in Form eines wasserlöslichen Salzes vorliegt und zusätzlich ein Tensid in wirksamer Menge anwesend ist. Besonders bevorzugt enthalten die Präparate ein Salz aus einem eine oder mehrere Carboxylgruppen aufweisenden Monoedelmetall-dithiolat und einem primären, sekundären oder tertiären Amin oder einer N-heterocyclischen Base. Der Anteil an organischen Lösungsmitteln beträgt vorzugsweise weniger als 1 Gew.-%, insbesondere 0 Gew.-%, bezogen auf das Präparat. Der genannte Restgehalt an organischen Lösungsmitteln kann beispielsweise aus der Verwendung der eingesetzten Hilfsstoffe resultieren, wenn diese in Form organischer Lösungen im Handel sind und so eingesetzt werden.

Die in wäßrig-organischen und rein wäßrigen erfindungsgemäßen Präparaten einsetzbaren Monoedelmetall-dithiolate, weisen Strukturelemente auf, nämlich mindestens eine zur Salzbildung befähigte saure oder basische Gruppe, welche ihnen eine ausreichende Wasserlöslichkeit vermitteln. Die Präparate enthalten im allgemeinen 2 bis 25 Gew.-%, vorzugsweise 5 bis 15 Gew.-%, Edelmetall in Form eines oder mehrerer Thiolate. Demgemäß muß die Wasserlöslichkeit des Thiolats bei hohem Edelmetallgehalt des Präparats höher sein als bei geringerem Gehalt. Angestrebt wird, ein organisches Dekor mit geringst möglicher Menge Gold zu erzielen.

Wäßrige erfindungsgemäße Präparate enthalten solche polymeren Bindemittel, welche in Wasser löslich sind oder darin eine klare Dispersion bilden. Zweckmäßige Bindemittel sind Polyacrylsäure, Polymethacrylsäure, Polyvinylpyrrolidon, Celluloseether, insbesondere Carboxyalkyl- und Hydroxyalkyl-Cellulose, Polyalkylenglykol, Polyvinylacetat, Polyvinylalkohol, Polyamine, Alkydharze und Polyurethanharze. Die Bindemittel können in Form von Homopolymeren oder Copolymeren oder Blockpolymeren jeweils einzeln oder in Form von Gemischen zur Anwendung gelangen. Besonders bevorzugt für rein wäßrige Präparate und solche mit hohem Wassergehalt werden Polyvinylpyrrolidon-homo- oder -copolymere, Polymethacrylsäure-homo- oder -copolymere sowie Harze auf der Basis von Celluloseethern.

Der Anteil an polymeren Bindemitteln in den organisch-wäßrigen und im wesentlichen rein wäßrigen Präparaten liegt üblicherweise im Bereich zwischen 3 und 45 Gew.-%, vorzugsweise zwischen 3 und 20 Gew.-% und insbesondere 4 bis 10 Gew.-%. Das Gewichtsverhältnis von Bindemittel zu Edelmetall in den Präparaten liegt zweckmäßigerweise im Bereich zwischen 0,1 bis 2, vorzugsweise zwischen 0,3 bis 1,2 und insbesondere zwischen 0,5 und 1,0. Der Wassergehalt wäßrig-organischer Präparate liegt im allgemeinen zwischen 10 und 80 Gew.-%, vorzugsweise zwischen 30 bis 60 Gew.-%, und der Gehalt an organischem Lösungsmittel zwischen 10 und 40 Gew.-%, jeweils bezogen auf das Präparat. Der Wassergehalt von im wesentlichen rein wäßrigen Präparaten liegt im allgemeinen zwischen 10 und 90 Gew.-%, vorzugsweise 40 bis 80 Gew.-%, jeweils bezogen auf das Präparat.

Präparate mit einem wäßrig-organischen Lösungsmittelsystem enthalten als organisches Lösungsmittel ein oder mehrere wasserlösliche organische Lösungsmittel, insbesondere wasserlösliche Alkohole, Ether und Ester. Besonders bevorzugt finden Glykole mit 2 bis 4 C-Atomen sowie Oligo- und Poly(C₂- bis C₄-)glykole oder Mono(C₁- bis C₄-)alkylether der genannten Glykole oder Oligoglykole, ferner (C₂- bis C₅-) Hydroxycarbonsäuren oder niedere Alkylester derselben, wie insbesondere C₁- bis C₃-Alkyllactat, Verwendung.

Erfindungswesentlicher Bestandteil der im wesentlichen rein wäßrigen Präparate ist ein mit dem Salz des Thiolats verträgliches Tensid aus der Reihe anionischer, nichtionischer, zwitterionischer und kationischer Tenside in wirksamer Menge. Die Verträglichkeit und Wirksamkeit eines ausgewählten Tensids wird der Fachmann durch orientierende Versuche überprüfen, weil Wechselwirkungen auch mit den anderen Bestandteilen des Präparate auftreten können. Üblicherweise wird mit einer Tensidmenge zwischen 0,1 und 2 Gew.-%, bezogen auf das Präparat, eine gute Wirkung erzielt; eine größere oder kleinere Menge ist im Einzelfall aber möglich. Vorzugsweise liegt der Tensidgehalt zwischen 0,2 und 1 Gew.-%.

Die Tenside enthalten im Molekül einen hydrophoben Rest von 8 bis 26 und insbesondere 10 bis 18 C-Atomen oder eine andere hydrophobe Gruppierung, wie beispielsweise eine solche auf der Basis Polydimethylsiloxan, und wenigstens eine anionische, zwitterionische, nichtionische oder kationische wasserlöslichmachende Gruppe.

Unter den anionischen Tensiden sind als geeignete Beispiele zu nennen: Geradkettige oder verzweigtkettige Alkylbenzolsulfonate, insbesondere solche mit einer geradkettigen C₈- bis C₁₆-Alkylgruppe, aliphatische und olefinische (C₈-C₁₈-)-Sulfonate, Hydroxyalkansulfonate, Fettsäureester der Oxyethansulfonsäure; Fettalkoholsulfate, sulfatierte Fettsäurealkylolamide und Fettsäuremonoglyceride sowie sulfatierte Alkoxylierungsprodukte von Fettalkoholen, Alkylphenolen, Fettsäureamiden; gesättigte und ungesättigte Fettsäuresalze, Alkyl- und Alkenyl-ethercarbonsäuresalze enthaltend eine (C₁₀-C₂₀-)-Alkyl- oder Alkenylgruppe und eine 1 bis 8 Einheiten umfassende Polyethylenglykolgruppe; α-Sulfofettsäuren; amidartige Kondensationsprodukte von Fettsäuren oder Sulfonsäuren mit Aminocarbonsäuren, wie Glycin, Sarkosin, Eiweishydrolysaten. Besonders geeignete anionische Tenside sind Alkylbenzolsulfonate mit 8 bis 14 C-Atomen, insbesondere in Form eines Salzes mit einem Amin in einer Menge von 0,1 bis 2 Gew.-%, vorzugsweise 0,2 bis 1 Gew.-%, bezogen auf das Präparat.

Nichtionische Tenside verdanken ihre Wasserlöslichkeit der Anwesenheit von Polyethergruppen, ferner Aminoxid-, Sulfoxid-, Phosphinoxid und Alkylolamidgruppierungen. Von besonderem Interesse sind Alkoxylierungsprodukte, besonders Ethoxylierungsprodukte von Fettalkoholen, Akylphenolen, Fettaminen, Alkanolaminen, Fettsäuren, Fettsäure- und Sulfonsäureamiden.

Eine weitere in den erfindungsgemäßen im wesentlichen rein wäßrigen Präparaten besonders wirksame Gruppe von nichtionischen Tensiden sind wasserlösliche polyethermodifizierte Polysiloxane - es handelt sich hierbei um Polyether-Polysiloxan-Copolymere, wobei lineare oder verzweigte Block-Copolymerstrukturen vorliegen können. Die Einsatzmenge liegt bei dieser Klasse vorzugsweise zwischen 0,1 und 2,0 Gew.-%, insbesondere zwischen 0,2 und 1,0 Gew.-%, bezogen auf das Präparat. Bei den Polyethersegmenten handelt es sich um Polyoxyethylen-, Polyoxypropylen- oder Poly(oxyethylen-oxypropylen)-Segmente, wobei Polyoxyethylensegmente und hierbei insbesondere solche mit 4 bis 20 Oxyethyleneinheiten bevorzugt werden. Die Polysiloxansegmente basieren vorzugsweise auf Dimethylsiloxan. Die Polyether- und Polysiloxansegmente können über Si-O-C- oder Si-C-Bindungen aneinander gebunden sein. Beispielhaft wird auf Ullmann's Encylopedia of Industrial Chemistry 5. ed., Vol. A 24, S. 83-84 (1993) und Ind. Eng. Chem. Proc. Res., Dev. 6 (1967), S. 88-92 verwiesen.

Zwitterionische Tenside enthalten sowohl eine saure, wie Carboxyl-, Sulfonsäure-, Schwefelsäurehalbester- oder Phosphorsäureteilestergruppe, als auch eine basische, wie, primäre, sekundäre, tertiäre, quaternäre Ammoniumgruppierung, hydrophile Gruppe. Betaine gehören hierzu, wie beispielsweise solche vom Typ 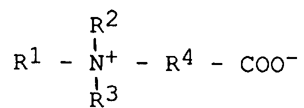 mit R¹ gleich Alkyl, Alkenyl, Hydroxyalkyl mit 8 bis 24 C-Atomen, R² und R³ gleich oder verschieden Alkyl oder Hydroxyalkyl mit 1 bis 4 C-Atomen, R⁴ gleich Alkylen oder Hydroxyalkylen mit 1 bis 6 C-Atomen.

Bei den kationischen Tensiden handelt es sich meist um Stoffe der Formel 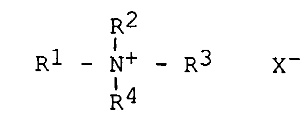 mit R¹ gleich Alkyl oder Alkenyl mit 8 bis 24 C-Atomen, R² bis R⁴ gleich Alkyl oder Hydroxyalkyl mit 1 bis 5 C-Atomen und X⁻ gleich ein Halogenatom.

Im Falle von Vergoldungspräparaten, insbesondere Glanzgoldpräparaten und Poliergoldpräparaten, enthalten diese zur Farbbeeinflussung eine geringe Menge einer oder mehrerer im System im wesentlichen löslicher anderer Edelmetallverbindungen in Form von Resinaten oder Sulforesinaten oder in Form einfacher Edelmetallsalze oder -komplexe. Zusätzlich enthalten die Präparate üblicherweise Flußmittel in Form von Verbindungen, beispielsweise Resinaten, Salzen, Oxiden oder Koordinationsverbindungen, eines oder mehrerer der Elemente Bor, Silicium, Vanadium, Chrom, Indium, Zinn, Antimon, Wismut und Rhodium. Die Einsatzmenge der Flußmittel bewegt sich üblicherweise im Bereich zwischen 0,01 und 2 Gew.-%, bezogen auf das Präparat.

Weitere Hilfsstoffe in den erfindungsgemäßen Präparaten können übliche Stoffe zur Veränderung der rheologischen Eigenschaften des Präparats, oberflächenaktive Mittel, die Haftung verstärkende Hilfsstoffe sowie Trocknungsbeschleuniger im Falle der Verwendung eines UV-härtbaren Harzes. Die zusätzliche Verwendung einer wäßrigen Polysulfidlösung hat sich in manchen Fällen, insbesondere in Polyvinylpyrrolidon enthaltenden Präparaten, als zweckmäßig erwiesen. Sogenannte Poliergoldpräparate enthalten im allgemeinen zusätzlich Goldpulver und/oder feinteilige unlösliche Goldverbindungen. Je nach gewünschtem Effekt können Poliergoldpräparate zusätzlich noch Glasfritten und/oder Organosiliciumverbindungen enthalten.

Zur Herstellung der wäßrigen Präparate wird unmittelbar ein wasserlösliches Salz eines Monoedelmetall-dithiolats eingesetzt oder das Salz wird bei Raumtemperatur oder erhöhter Temperatur, vorzugsweise bei 30 bis 80 °C in Wasser in situ gebildet, aus einem mindestens eine saure funktionelle Gruppe aufweisenden Monoedelmetall-dithiolat und einer Base, etwa einer Alkalilauge, Ammoniak oder einem Amin oder aus einem eine Aminogruppe aufweisenden Monoedelmetall-dithiolat und einer Säure, wie niederen Carbonsäure.

Pro Mol Thiolat wird im allgemeinen die zur Neutralisation der sauren Gruppen erforderliche Menge Base eingesetzt; in einigen Fällen wird bereits bei einem Baseunterschuß eine vollständige Lösung erzielt. Ein Baseüberschuß ist möglich, im allgemeinen aber nicht erforderlich. Zur erhaltenen Lösung werden die Flußmittel gegeben und gelöst. Das Bindemittel wird anschließend unmittelbar in die Lösung eingetragen und darin aufgelöst. Alternativ hierzu kann das Bindemittel oder Bindemittelgemisch, ggf. unter Zusatz weiterer Hilfsstoffe, wie etwa einer Polysulfidlösung, in Wasser aufgelöst und diese Lösung mit der edelmetallhaltigen Lösung vereinigt werden. Das Tensid wird während der Präparatherstellung einer der Lösungen oder am Ende der Herstellung dem Präparat selbst zugesetzt.

Die erfindungsgemäßen Monoedelmetall-dithiolate enthaltenden Präparate können unmittelbar mittels üblicher Dekorationsverfahren, wie Sprühen, Pinseln oder bekannter Druckverfahren, insbesondere Siebdruckverfahren, auf die zu dekorierende Oberfläche aufgebracht und nach dem Abdunsten der Lösungsmittelbestandteile das Dekor bei einer Temperatur zwischen im allgemeinen 400 und 900 °C eingebrannt werden. Alternativ läßt sich das erfindungsgemäße Edelmetallpräparat unter Mitverwendung eines Abziehbilds auf den zu dekorierenden Gegenstand übertragen. Die Herstellung des Abziehbilds erfolgt in für den Fachmann bekannter Weise, wobei sich die erfindungsgemäße Monogold(I)-dithiolatverbindung in der Dekorschicht des Abziehbilds befindet: Auf eine auf einen Träger aufgebrachte wasserlösliche Trennschicht oder eine Thermotrennschicht wird unter Verwendung eines zuvor beschriebenen edelmetallhaltigen Präparats und Verdunstenlassen der Lösungsmittelbestandteile und gegebenenfalls Vernetzen des Bindemittels eine Dekorschicht aufgebracht; die Dekorschicht wird üblicherweise überfilmt.

Durch die Erfindung wurde eine neue Stoffklasse zur Verfügung gestellt, welche sich hervorragend zur Herstellung von filmartigen edelmetallhaltigen Dekoren eignen. Die Dekore sind überraschend hochglänzend und haften ausgezeichnet auf der Substratfläche, da ein zusammenhängender Film gebildet wird. Durch die erfindungsgemäßen Monoedelmetall-dithiolate wird der Fachwelt eine neue Stoffklasse zur Verfügung gestellt, welche nicht nur in konventionellen Edelmetallpräparaten, welche als Lösungsmittel im wesentlichen nur organische Lösungsmittel enthalten, eingesetzt werden können, sondern vielmehr in solchen Edelmetallpräparaten, welche ein wäßrig-organisches Lösungsmittelsystem oder im wesentlichen nur Wasser als Lösungsmittel enthalten. Die Herstellung neuer Edelmetallverbindungen sowie deren herausragende Eignung in wasserhaltigen und rein wäßrigen Präparaten für die Dekoration einbrennfähiger Substrate folgt aus den Beispielen. Die Vergleichsbeispiele zeigen, daß unter Verwendung strukturähnlicher Verbindungen, welche keine zweite Mercaptogruppe enthalten, keine oder weniger befriedigende Edelmetalldekore hergestellt werden können.

Die mittels erfindungsgemäßer Präparate hergestellten Dekore sind überraschend hochglänzend und haften ausgezeichnet auf der Substratfläche, da ein zusammenhängender Film gebildet wird. Zudem gelingt es, flecken- und porenfreie Filme zu erzeugen, welche den ästhetischen Erfordernissen entsprechen.

Im Hinblick auf den Stand der Technik war nicht vorhersehbar, daß es möglich ist, Edelmetallpräparate zur Herstellung von Edelmetalldekoren, insbesondere Glanzgolddekoren, zur Verfügung zu stellen, in welchen auf den Einsatz von organischen Lösungsmitteln verzichtet werden kann. Durch die Kombination von speziellen erfindungsgemäßen Thiolaten, wasserlöslichen Polymeren und einem Tensid ist es, wie die nachfolgenden Beispiele zeigen, möglich, rein wäßrige Präparate zu erhalten und mit ihrer Hilfe hochwertige Dekore auf einbrennfähigen Substraten herzustellen.

### Beispiel 1

### Herstellung von Monogold-dimercaptobernsteinsäure

a) 0,035 mol = 18,49 g HAuCl₄ (37,29 % Au) wird unter Rühren zu einer Suspension von 0,07 mol Methionin in 70 ml H₂O getropft. Durch externe Kühlung wird die Temperatur auf 0 bis 5 °C gehalten. Nach Beendigung der Reaktion wird der Gold(I)-Komplex zu einer Suspension von 0,035 Mol = 6,38 g meso-2,3-Dimercaptobernsteinsäure in 150 ml Dichlormethan innerhalb einer Stunde getropft. Der Niederschlag wird abgesaugt, mehrmals mit Wasser gewaschen und im Exikkator unter Vakuum über Blaugel getrocknet. Die Ausbeute an Monogolddimercaptobernsteinsäure beträgt 96,5 %, bezogen auf das eingesetzte Gold.

| Analyse | Au | C | H | S |
|---|---|---|---|---|
| berechnet | 52,09 % | 12,70 % | 1,33 % | 16,96 % |
| gefunden | 50,81 % | 12,05 % | 1,52 % | 15,62 % |
| Zersetzungsbeginn: 194 °C | | | | |

13_{C-NMR} in D₂O + Trimethylamin δ/ppm 52,58 (CH), 60,95 (CH), 180,64 (CO₂⁻) und 182,51 (CO₂⁻)
b) Das Beispiel 1a wurde wiederholt, wobei jedoch anstelle Dichlormethan Wasser verwendet wurde. Auch in diesem Fall wurde Monogold(I)-dimercaptobernsteinsäure in hoher Ausbeute und Reinheit gewonnen.

### Vergleichsbeispiel 1

Analog Vergleichsbeispiel 7 in der EP-A 0 514 073 werden folgende Bestandteile gemischt:

| | Gewichtsteile |
|---|---|
| Polymethacrylatharz (Versicol K11 von Allied Colloids)) | 11 |
| Goldmercaptobernsteinsäure | 18 |
| 1,3-Propandiol | 15 |
| Wasser | 40 |
| Isopropanol | 15 |
| Rhodium-Komplex | 0,04 |
| Chromtrioxid | 0,05 |

Das Präparat wird auf Porzellan gepinselt und 10 min bei 820 °C erhitzt, wobei die Aufheizzeit eine Stunde beträgt. Man erhält einen matten Film. Glanzmessungen ergaben folgende Werte:

| Winkel | Glanzeinheiten | Standardabweichung |
|---|---|---|
| 20 ° | 58,3 | 5,4 |
| 60 ° | 54,2 | 4,1 |

Die Glanzmessungen wurden mit dem Reflekometer "haze-gloss" der Fa. ByK Gardner durchgeführt. Das Präparat wurde auf eine Porzellanplatte 4 cm x 4 cm aufgepinselt und an verschiedenen Stellen gemessen. Angegeben wird der Mittelwert der einzelnen Messungen und die dazugehörige Standardabweichung, wie sie vom Meßgerät angegeben wurde, sowie der Winkel, unter dem die Messungen durchgeführt wurden.

### Vergleichsbeispiel 2

Die folgende Zusammensetzung wird analog dem Vergleichsbeispiel 1 präpariert:

| | Gewichtseile |
|---|---|
| Polyvinylpyrrolidon (PVP K25 von Fluka) | 8,1 |
| Goldmercaptobernsteinsäure | 17,2 |
| 1,2-Propandiol | 8,1 |
| Wasser | 39,9 |
| Ethyllactat | 16,2 |
| Rhodium-Komplex | 0,15 |
| Chromsulfat | 0,04 |
| Ammoniumwismutcitrat | 0,44 |
| Triethylamin | 5,3 |
| Ammoniumpolysulfidlösung (15 %ig in Wasser) | 1,9 |
| Silbermercaptopropionylglycin | 2,6 |

Das Präparat wird auf Porzellan gepinselt und 10 min bei 820 °C erhitzt, wobei die Aufheizzeit eine Stunde beträgt. Man erhält einen matten Film. Glanzmessungen ergaben folgende Werte:

| Winkel | Glanzeinheiten | Standardabweichung |
|---|---|---|
| 20 ° | 20,1 | 4,6 |
| 60 ° | 38,2 | 2,1 |

### Beispiel 2

In der folgenden Zusammensetzung wurde im Unterschied zum Vergleichsbeispiel 2 die erfindungsgemäße Goldverbindung gemäß Beispiel 1 eingesetzt:

| | Gewichtsteile |
|---|---|
| PVP K25 | 8,1 |
| Monogolddimercaptobernsteinsäure | 18,8 |
| 1,2-Propandiol | 8,1 |
| Wasser | 38,3 |
| Ethyllactat | 16,2 |
| Rhodium-Komplex | 0,15 |
| Chromsulfat | 0,04 |
| Ammoniumwismutcitrat | 0,44 |
| Triethylamin | 5,3 |
| Ammoniumpolysulfidlösung (15 %ig) | 1,9 |
| Silbermercaptopropionylglycin | 2,6 |

Das Präparat wird auf Porzellan gepinselt und 10 min bei 820 °C erhitzt, wobei die Aufheizzeit eine Stunde beträgt. Man erhält einen glänzenden, gut haftenden Film. Glanzmessungen ergaben folgende Werte:

| Winkel | Glanzeinheiten | Standardabweichung |
|---|---|---|
| 20 ° | 741 | 42,4 |
| 60 ° | 503 | 6,4 |

### Beispiel 3

Folgende Bestandteile wurden gemischt:

| | Gewichtsteile |
|---|---|
| PVP K25 | 8,6 |
| Monogolddimercaptobernsteinsäure | 19,5 |
| 1,2-Propandiol | 8,6 |
| Wasser | 34,3 |
| Ethyllactat | 17,3 |
| Rhodium-Komplex | 0,16 |
| Chromsulfat | 0,07 |
| Ammoniumwismutcitrat | 0,53 |
| Triethylamin | 5,7 |
| Ammoniumpolysulfidlösung (15 %ig) | 2,6 |
| Monosilberdimercaptobernsteinsäure | 2,6 |

Das Präparat auf das Porzellan gepinselt 2 min bei 880 °C erhitzt, wobei die Aufheizzeit 30 min beträgt. Man erhält einen glänzenden, gut haftenden Film.

### Beispiele 4 bis 8

Analog zur in Beispiel 1 angegebenen Herstellungsweise wurden weitere Monogold-dimercaptoverbindungen durch Umsetzung des Gold(I)-methioninkomplexes mit weiteren Dimercaptoverbindungen im Molverhältnis 1 zu 1 hergestellt:
- Beispiel 4:: Umsetzung mit 1,4-Dimercapto-2,3-butandiol: Der Au-Gehalt des isolierten Produktes betrug 67,01 % (theor. 56,24).
- Beispiel 5:: Umsetzung mit 2,3-Dimercapto-1-propansulfonsäure-Natriumsalz: Au-Gehalt 57,45 % (theor. 46,43 %). Das erhaltene, im wesentlichen aus der Monogoldverbindung und teilweise offensichtlich auch der Digoldverbindung der angegebenen Sulfonsäure bestehende Produkt löste sich glatt in Wasser. Das Produkt eignet sich zur Herstellung von Edelmetalldekoren, insbesondere solchen, welche nach dem Polieren einen seidenmatten Glanz geben.
- Beispiel 6:: Umsetzung mit 6,8-Dimercaptooctansäure: Au-Gehalt 57,47 % (theor. 48,72 %). Die Verbindung ist als Goldpräparat in organischen Dekorpräparaten, wegen der geringen Wasserlöslichkeit aber nicht in wäßrigen Präparaten geeignet.
- Beispiel 7:: Umsetzung mit N-(3'-Mercapto-2'-methylpropionyl)2-amino-4-mercapto-butansäure: Au-Gehalt 51,22 % (theor. 45,46 %). Die erhaltene Monogolddimercaptoverbindung eignet sich als Au-Verbindung in organischen und wäßrigen Dekorpräparaten, welche schön ausbrennen und ein glänzendes und helles Dekor ergeben.
- Beispiel 8:: Umsetzung mit α,α'-Dimercaptoadipinsäure: Au-Gehalt 49,07 % (theor. 48,48 %). Die Au-Verbindung brennt in Dekorpräparaten zunächst etwas matt aus, nach leichtem Polieren wird aber ein sehr schöner heller Glanz erzielt. Die Au-Verbindung eignet sich damit besonders in niedrig-Au-prozentigen Poliergoldpräparaten. Der niedrige Au-Gehalt ist ein Vorteil im Vergleich zu üblichen Poliergoldpräparaten.

### Beispiel 9

### Glanzgoldpräparat

Gemäß allgemeiner Arbeitsvorschrift wurde eine wäßrige Lösung hergestellt, welche Monogold(I)-dimercaptobernsteinsäure enthielt. Als Base wurde Triethylamin eingesetzt. Als Flußmittel wurden dann Rhodiumchlorid, Chromsulfat und Ammoniumwismutcitrat zugesetzt. Zur erhaltenen Lösung wurde eine wäßrige Polyvinylpyrrolidonlösung, welche zusätzlich Ammoniumpolysulfid enthielt, zugegeben, ferner als Tensid ein polyethermodifiziertes Polydimethylsiloxan (ByK®346). Das Präparat enthielt nur etwa 0,5 Gew.-% eines in der handelsüblichen Tensidlösung enthaltenen organischen Lösungsmittels und war ansonsten rein wäßrig.

Die Edelmetall- und Flußmittelgehalte, berechnet wie angegeben, sowie der Gehalt an Bindemittel, Tensid und Hilfsstoffen im Präparat folgen aus der Tabelle.

| Bestandteile | Gew.-% im Präparat |
|---|---|
| Au-Gehalt | 9,35 |
| Rh-Gehalt | 0,06 |
| Cr₂O₃-Gehalt | 0,13 |
| Bi₂O₃-Gehalt | 0,21 |
| Polyvinylpyrrolidon (PVP K25 von Fluka) | 5,2 |
| Tensidlösung (ByK®346; Tensidgehalt 46 %) | 1,0 |
| N(C₂H₅)₃ (zur Salzbildung) | 4,8 |
| (NH₄)₂ Sx | 0,15 |

### Beispiele 10 und 11

Analog Beispiel 9 wurden im wesentlichen rein wäßrige Glanzgoldpräparate hergestellt, wobei als Au-Thiolat das Produkt aus Beispiel 1 und zusätzlich Silber-2-mercaptopropionylglycin als Silberthiolat zum Einsatz kamen. Als Bindemittel diente eine Hydroxyethylcellulose (Natrasol von Aqualon Ltd., UK); Polysulfid kam nicht zum Einsatz. Im erfindungsgemäßen Präparat gemäß Beispiel 10 war als Tensid wiederum ByK®346 zugegen; das nichterfindungsgemäße Präparat gemäß Beispiel 11 enthielt kein Tensid. Die Gehalte der Bestandteile folgen aus der Tabelle.

| Bestandteile | Beispiel 10 | Beispiel 11 |
|---|---|---|
| | (Gew.-% im Präparat) | |
| Au-Gehalt | 9,62 | 9,71 |
| Ag-Gehalt | 1,08 | 1,10 |
| Rh-Gehalt | 0,07 | 0,07 |
| Cr₂O₃-Gehalt | 0,01 | 0,01 |
| Bi₂O₃-Gehalt | 0,30 | 0,30 |
| Hydroxyethylcellulose | 5,3 | 5,3 |
| Tensidlösung (ByK®346; Tensidgehalt 46 %) | 1,0 | - |
| N(C₂H₅)₃ (zur Salzbildung) | 5,3 | 5,3 |

### Beispiel 12

Dekoration von Porzellan

Auftrag der Präparate der Beispiele 9, 10 und 11 mittels Siebdruck (Beispiel 12/9, 12/10, 12/11).

Brennbedingungen: Aufheizen auf 820 °C in 1 h, Haltezeit bei 820 °C 10 Minuten.

Unter Verwendung der erfindungsgemäßen Präparate der Beispiele 9 und 10 wurden glänzende und fleckenfreie, gut haftende Dekorfilme erhalten. Unter Verwendung des tensidfreien, nicht-erfindungsgemäßen Präparats von Beispiel 11 wurden nur fleckige Dekore erhalten.

## Patentansprüche

1. Monoedelmetall-dithiolat der allgemeinen Formel (A) 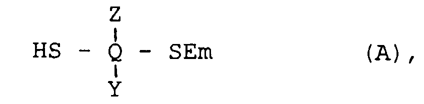 worin bedeuten
Em: ein Edelmetall aus der Reihe Au (I) oder Ag (I) oder ein Äquivalent von Pd(II), Pt(II) oder Rh(III)
Q: ein tetravalenter organischer Rest mit 2 bis 10 C-Atomen
Y: eine hydrophile Gruppe aus der Reihe -COOH, -COO⁻Kat⁺, wobei Kat⁺ für Li⁺, Na⁺, K⁺, NH₄⁺, (C₁- bis C₃-Alkyl)ₙ N⁺H₍₄₋ₙ₎ oder (Hydroxy-(C₁- bis C₃-)alkyl)ₙ N⁺H₍₄₋ₙ₎ und n für eine ganze Zahl zwischen 1 und 4 stehen, oder 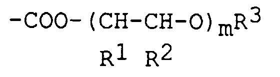 mit R¹, R² und R³ gleich oder verschieden H oder Methyl und m gleich eine ganze Zahl von 1 bis 12,
Z: gleich Y oder -H oder eine Gruppe aus der Reihe -OR', -SR', -SO₃R', -NR'₂, NR'₃⁺X⁻, wobei X⁻ für ein Mineralsäure- oder Carbonsäureanion und R' für Wasserstoff oder die Gruppe 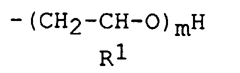 stehen und R¹ und m die vorgenannte Bedeutung haben.

2. Monoedelmetall-dithiolat nach Anspruch 1,
dadurch gekennzeichnet,
daß Em für Gold oder Silber, jeweils in der Oxidationsstufe (I), steht.

3. Monoedelmetall-dithiolat nach Anspruch 1 oder 2,
dadurch gekennzeichnet,
daß dieses wasserlöslich ist und Y und/oder Z COO⁻Kat⁺ bedeuten oder Y eine Carboxylgruppe und Z eine zur inneren Salzbildung mit der Carboxylgruppe befähigte Aminogruppe der Formel -NR'₂, wobei Kat⁺ und R' die vorgenannte Bedeutung haben.

4. Monoedelmetall-dithiolat nach einem der Ansprüche 1 bis 3,
dadurch gekennzeichnet,
daß der organische Rest Q ein aliphatischer C₂- bis C₄-Alkantetraylrest oder ein 5- oder 6-gliedriger, gegebenenfalls ein Sauerstoff- oder Imin-Ringglied enthaltender cycloaliphatischer Tetraylrest ist.

5. Monoedelmetall-dithiolat nach einem der Ansprüche 1 bis 4,
dadurch gekennzeichnet,
daß es Monogold(I)- oder Monosilberdimercaptobernsteinsäure oder ein Salz derselben mit dem Kation Kat⁺ mit der vorgenannten Bedeutung ist.

6. Verfahren zur Herstellung von Monoedelmetall-dithiolaten der allgemeinen Formel (A') 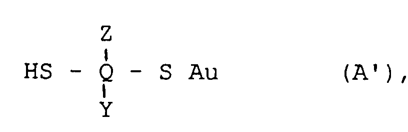 worin Q, Z und Y die Bedeutung gemäß Anspruch 1 haben, durch Umsetzung eines Au(I)-Komplexes der Formel AuCl·R''SR''', wobei R''SR''' ein Thioether, vorzugsweise Methionin ist, mit einer im wesentlichen äquivalenten Molmenge eines Thiols in Gegenwart eines Lösungsmittels,
dadurch gekennzeichnet,
daß man als Thiol ein solches der allgemeinen Formel (B) 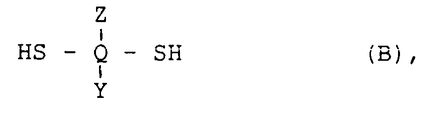 wobei Q, Y und Z die gleiche Bedeutung haben wie in der Formel (A'), einsetzt.

7. Verfahren zur Herstellung von Monoedelmetall-dithiolaten der Formel A gemäß Anspruch 1, wobei Em für Ag(I) oder ein Äquivalent Pd(II) oder Pt(II) steht, durch Umsetzung eines löslichen Salzes von Ag(I), Pd(II) oder Pt(II) mit einer Thiolverbindung,
dadurch gekennzeichnet,
daß man als Thiolverbindung eine Dithiolverbindung der Formel B gemäß Anspruch 6 einsetzt.

8. Edelmetallhaltiges Präparat zum Dekorieren einbrennfähiger Unterlagen, insbesondere Glas, Porzellan und Keramik, zwecks Herstellung eines Edelmetalldekors, insbesondere hochglänzenden Edelmetalldekors, enthaltend ein oder mehrere Edelmetallthiolate, ein oder mehrere polymere organische Bindemittel, ferner Lösungsmittel für das/die Edelmetall-thiolat/e und das/die Bindemittel und, soweit erforderlich, Hilfsstoffe zur Einstellung der Verarbeitungseigenschaften des Präparates und der optischen und Gebrauchseigenschaften des damit herzustellenden edelmetallhaltigen Dekors,
dadurch gekennzeichnet,
daß es mindestens ein Monoedelmetall-dithiolat der Ansprüche 1 bis 5 als Edelmetallthiolat enthält.

9. Präparat nach Anspruch 8,
dadurch gekennzeichnet,
daß es als Lösungsmittel ein polares organisches Lösungsmittel, insbesondere aus der Reihe der C₂- bis C₄-Glykole, Oligo(C₂- bis C₄)glykole, C₁- bis C₄-Monoether der genannten Glykole und Oligoglykole, (C₂- bis C₃-)Hydroxycarbonsäuren und (C1- bis C3-)Alkylester derselben, und Wasser enthält.

10. Edelmetallhaltiges Präparat nach Anspruch 8,
dadurch gekennzeichnet,
daß es als Lösungsmittel Wasser und weniger als 2 Gew.-%, bezogen auf das Präparat, organische Lösungsmittel enthält, das Bindemittel wasserlöslich ist, das Monoedelmetall-dithiolat mindestens eine zur Salzbildung befähigte funktionelle Gruppe aufweist und in Form eines wasserlöslichen Salzes vorliegt und zusätzlich ein Tensid in wirksamer Menge anwesend ist.

11. Präparat nach einem der Ansprüche 8 bis 10,
dadurch gekennzeichnet,
daß das polymere organische Bindemittel ausgewählt ist aus der Reihe der Polyvinylpyrrolidon-homo- oder copolymeren, Polyacrylsäurehomo- oder -copolymeren, Polymetharylsäurehomo- oder -copolymeren und wasserlöslichen Celluloseether sowie Gemischen der genannten Bindemittel.

12. Präparat nach Anspruch 10 oder 11,
dadurch gekennzeichnet,
daß es ein wasserlösliches Salz aus einem eine oder mehrere Carboxylgruppen aufweisenden Monoedelmetall-dithiolat und einem primären, sekundären oder tertiären Amin oder einer N-heterocyclischen Base enthält.

13. Präparat nach einem der Ansprüche 10 bis 12,
dadurch gekennzeichnet,
daß das Monoedelmetall-dithiolat in Form eines Salzes mit Tri(C₁- bis C₃)alkylamin oder Mono-, Di- oder Triäthanolamin vorliegt und das Präparat zusätzlich übliche Hilfsstoffe zur Einstellung der optischen und Gebrauchseigenschaften des herzustellenden Dekors und/oder der Verarbeitungseigenschaften des Präparats enthält.

14. Präparat nach einem der Ansprüche 8 bis 13,
dadurch gekennzeichnet,
daß es ein wasserlösliches Salz der Monogold(I)-dimercaptobernsteinsäure enthält.

15. Präparat nach einem der Ansprüche 10 bis 14,
dadurch gekennzeichnet,
daß es als Tensid 0,1 bis 2,0 Gew.-%, vorzugsweise 0,2 bis 1,0 Gew.-%, eines Polyoxyethylenpolydimethylsiloxans enthält.

16. Präparat nach einem der Ansprüche 10 bis 15,
dadurch gekennzeichnet,
daß es als Tensid ein Alkylbenzolsulfonat mit 8 bis 14 C-Atomen in der Alkylgruppe in Form eines Salzes mit einem Amin in einer Menge von 0,1 bis 2 Gew.-%, vorzugsweise 0,2 bis 1 Gew.-%, enthält.

17. Abziehbild zur Herstellung edelmetallhaltiger Dekore auf einbrennfähigen Unterlagen, insbesondere Glas, Porzellan und Keramik, enthaltend in einer Dekorschicht ein oder mehrere Edelmetallthiolate, ein oder mehrere polymere organische Bindemittel und bei Bedarf Hilfsstoffe aus der Herstellung des Abziehbilds und zur Beeinflussung der optischen und Gebrauchseigenschaften des herzustellenden edelmetallhaltigen Dekors,
dadurch gekennzeichnet,
daß ein Monoedelmetall-dithiolat gemäß einem der Ansprüche 1 bis 5 in homogener Verteilung als Edelmetallthiolat anwesend ist.

18. Verfahren zum Dekorieren einbrennfähiger Substrate, insbesondere Glas, Porzellan oder Keramik durch Aufbringen eines edelmetallhaltigen Präparats oder eines ein solches Präparat in getrocknetem Zustand enthaltenden Abziehbilds auf das Substrat und Einbrennen bei 400 bis 900 °C,
dadurch gekennzeichnet,
daß man ein edelmetallhaltiges Präparat gemäß einem der Ansprüche 8 bis 16 oder ein Abziehbild gemäß Anspruch 17 aufbringt.

## Claims

1. Mono-noble metal dithiolate of the general formula (A) 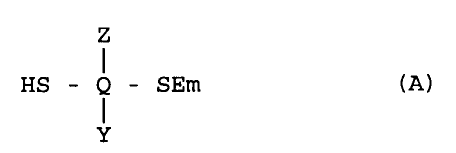 in which
Em: denotes a noble metal from the series Au(I) or Ag (I) or an equivalent of Pd(II), Pt(II) or Rh(III)
Q: denotes a tetravalent organic radical having 2 to 10 C atoms
Y: denotes a hydrophilic group from the series -COOH, -COO⁻cat⁺, wherein cat⁺ stands for Li⁺, Na⁺, K⁺, NH₄⁺, (C₁ to C₃ alkyl)ₙ N⁺H₍₄₋ₙ₎ or (hydroxy-(C₁ to C₃)alkyl)ₙ N⁺H₍₄₋ₙ₎ and n stands for an integer between 1 and 4, or 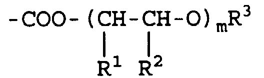 having R¹, R² and R³, which are the same or different, H or methyl and having m equal to an integer from 1 to 12,
Z: is equal to Y or -H or a group from the series -OR', -SR', -SO₃R', -NR'₂, NR'₃⁺X⁻, wherein X⁻ stands for a mineral acid anion or carboxylic acid anion and R' stands for hydrogen or the group 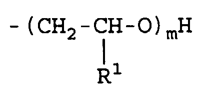 and R¹ and m denote the same as hereinabove.

2. Mono-noble metal dithiolate according to Claim 1, characterised in that Em stands for gold or silver, in each case in the oxidation state (I).

3. Mono-noble metal dithiolate according to Claim 1 or 2, characterised in that it is water-soluble, and Y and/or Z denote COO⁻cat⁺ or Y denotes a carboxyl group and Z an amino group of the formula -NR'₂ rendered capable of internal salt formation with the carboxyl group, wherein cat⁺ and R' denote the same as hereinabove.

4. Mono-noble metal dithiolate according to one of Claims 1 to 3, characterised in that the organic radical Q is an aliphatic C₂ to C₄ alkane tetrayl radical or a 5- or 6-membered cycloaliphatic tetrayl radical optionally containing an oxygen or an imine in the ring.

5. Mono-noble metal dithiolate according to one of Claims 1 to 4, characterised in that it is monogold(I) dimercaptosuccinic acid or monosilver dimercaptosuccinic acid or a salt thereof with the cation cat⁺ denoting the same as hereinabove.

6. Process for the preparation of mono-noble metal dithiolates of the general formula (A') 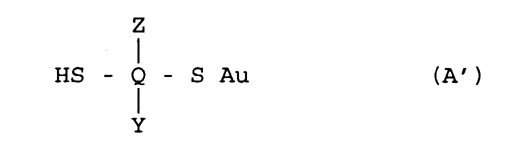 in which Q, Z and Y denote the same as in Claim 1, by reacting an Au(I) complex of the formula Aucl · R"SR"', wherein R"SR"' is a thioether, preferably methionine, with a substantially equivalent molar quantity of a thiol in the presence of a solvent, characterised in that the thiol used is a thiol of the general formula (B) 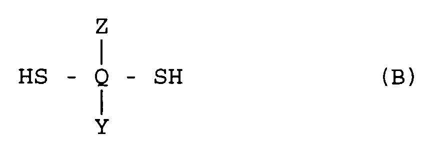 wherein Q, Y and Z denote the same as in the formula (A').

7. Process for the preparation of mono-noble metal dithiolates of the formula A according to Claim 1, wherein Em stands for Ag(I) or an equivalent of Pd(II) or Pt(II), by reacting a soluble salt of Ag(I), Pd(II) or Pt(II) with a thiol compound, characterised in that the thiol compound used is a dithiol compound of the formula B according to Claim 6.

8. Noble metal-containing preparation for decorating substrates capable of being fired, in particular glass, porcelain and ceramic, for the purpose of producing a noble metal decoration, in particular a high-gloss noble metal decoration, containing one or more noble metal thiolates, one or more polymeric organic binders, furthermore solvent for the noble metal thiolate(s) and the binder(s) and, where necessary, auxiliary substances for adjusting the processing characteristics of the preparation and the optical and service characteristics of the noble metal-containing decoration to be produced therewith, characterised in that it contains as the noble metal thiolate at least one mono-noble metal dithiolate of the Claims 1 to 5.

9. Preparation according to Claim 8, characterised in that it contains as the solvent a polar organic solvent, in particular from the series C₂ to C₄ glycols, oligo(C₂ to C₄) glycols, C₁ to C₄ monoethers of the named glycols and oligoglycols, (C₂ to C₃) hydroxycarboxylic acids and (C₁ to C₃ ) alkyl esters thereof, and water.

10. Noble metal-containing preparation according to Claim 8, characterised in that it contains as the solvent water and less than 2 wt.%, calculated on the preparation, of organic solvent, the binder is water-soluble, the mono-noble metal dithiolate exhibits at least one functional group rendered capable of forming salt and is present in the form of a water-soluble salt, and there is present additionally an effective quantity of a surfactant.

11. Preparation according to one of Claims 8 to 10, characterised in that the polymeric organic binder is selected from the series of polyvinyl pyrrolidone homopolymers or copolymers, polyacrylic acid homopolymers or copolymers, polymethacrylic acid homopolymers or copolymers and water-soluble cellulose ethers and mixtures of the named binders.

12. Preparation according to Claim 10 or 11, characterised in that it contains a water-soluble salt of a mono-noble metal dithiolate exhibiting one or more carboxyl groups and a primary, secondary or tertiary amine or an N-heterocyclic base.

13. Preparation according to one of Claims 10 to 12, characterised in that the mono-noble metal dithiolate is present in the form of a salt with tri(C₁ to C₃) alkylamine or mono, di or triethanolamine, and the preparation additionally contains conventional auxiliary substances for adjusting the optical and service characteristics of the decoration to be produced and/or the processing characteristics of the preparation.

14. Preparation according to one of Claims 8 to 13, characterised in that it contains a water-soluble salt of monogold(I) dimercaptosuccinic acid.

15. Preparation according to one of Claims 10 to 14, characterised in that it contains as the surfactant from 0.1 to 2.0 wt.%, preferably from 0.2 to 1.0 wt.%, of a polyoxyethylene polydimethyl siloxane.

16. Preparation according to one of Claims 10 to 15, characterised in that it contains as the surfactant a quantity of from 0.1 to 2 wt.%, preferably from 0.2 to 1 wt.%, of an alkylbenzene sulphonate having 8 to 14 C atoms in the alkyl group in the form of a salt with an amine.

17. Transfer picture for producing noble metal-containing decorations on substrates capable of being fired, in particular glass, porcelain and ceramic, containing in a decoration layer one or more noble metal thiolates, one or more polymeric organic binders and, if required, auxiliary substances from the production of the transfer picture and for influencing the optical and service characteristics of the noble metal-containing decoration to be produced, characterised in that there is present in homogeneous distribution as the noble metal thiolate a mono-noble metal dithiolate according to one of Claims 1 to 5.

18. Process for decorating substrates capable of being fired, in particular glass, porcelain or ceramic, by applying to the substrate a noble metal-containing preparation or a transfer picture containing such a preparation in the dried state, and firing at from 400 to 900°C, characterised in that there is applied a noble metal-containing preparation according to one of Claims 8 to 16 or a transfer picture according to Claim 17.

## Revendications

1. Dithiolate de mono(métal précieux) de formule générale (A), 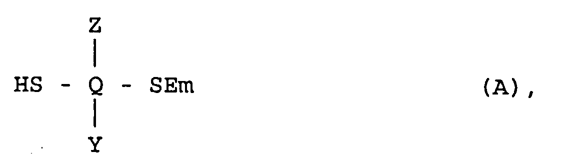 où
Em représente un métal précieux de la série de Au (I) ou un équivalent de Pd (II), Pt (II) ou Rh (II),
Q est un reste organique tétravalent de 2 à 10 atomes de C,
Y est un groupe hydrophile de la série -COOH, -COO Kat⁺, où Kat⁺ représente LI⁺, Na⁺, NH₄⁺, (alkyl C₁ à C₃)ₙ NH⁺₍₄₋ₙ) ou (hydroxy-(alkyl C₁ à C₃)ₙN⁺H₍₄₋ₙ₎ et n est un nombre entier entre 1 et 4, ou 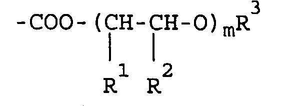 ou R¹, R² et R³ sont identiques ou différents et représentent H ou un méthyle et m est un nombre entier de 1 à 12, Z est identique à Y ou H ou à un groupe de la série -OR¹, -SR¹, -SR¹, -SO₃R¹, -NR'₂, NR'₃⁺X⁻, ou X⁻ représente un acide minéral ou un anion acide carboxylique et R¹ est l'hydrogène ou le groupe 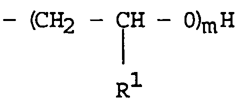 et R¹ et m ont la signification ci-dessus.

2. Dithiolate de mono(métal précieux) selon la revendication 1,
caractérisé en ce que
Em représente l'or ou l'argent, respectivement à l'état d'oxydation (I).

3. Dithiolate de mono(métal précieux) selon la revendication 1 ou 2,
caractérisé en ce qu'
il est hydrosoluble et que Y et/ou Z représentent COO⁻Kat⁺ ou Y est un groupe carboxyle et Z un groupe amino de formule -NR'₂ permettant la formation d'un sel avec le groupe carboxyle, où Kat⁺ et R' ont la signification déjà citée.

4. Dithiolate de mono(métal précieux) selon l'une des revendications 1 à 3
caractérisé en ce que
le radical organique Q est un radical alcane tétrayle ou un reste tétrayle cycloaliphatique de 5 ou 6 maillons, le cas échéant contenant un maillon de cycle imine ou oxygène.

5. Dithiolate de mono(métal précieux) selon l'une des revendications 1 à 4
caractérisé en ce qu'
il est un acide mono-or (I) ou mono-argent-dimercaptosuccinique ou un de ses sels du cation Kat ayant la signification déjà donnée ci-dessus.

6. Procédé de préparation de Dithiolates de mono(métal précieux) de formule générale (A'). 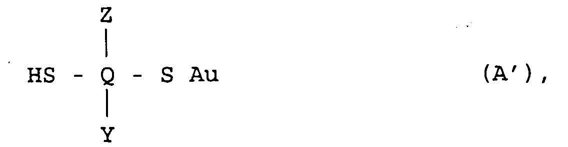 où
Q, Y et Z ont la signification selon la formule 1, par réaction d'un complexe Au (I) de formule AuCl R"SR"', ou (R"SR"'), est un thioéther, de préférence la méthionine, avec une quantité essentiellement équivalente molaire d'un thiol en présence d'un solvant
caractérisé en ce qu'
on utilise comme thiol un thiol de formule générale (B), 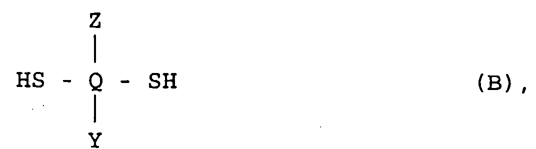 où Q, Y et Z ont la même signification que pour la formule (A').

7. Procédé de préparation de Dithiolates de mono(métal précieux) de formule A selon la revendication 1, où Em représente Ag (I) ou un équivalent de Pd (II) ou Pt (II), par réaction d'un sel soluble de Ag (I), Pd (II) ou Pt (II) avec un composé thiol,
caractérisé en ce qu'
on utilise comme composé thiol un composé dithiol de formule B selon la revendication 6.

8. Préparation de métal précieux pour décorer des supports à cuire, notamment du verre, de la porcelaine et de la céramique, pour produire un décor de métal précieux, notamment un décor de métal précieux très brillant contenant un ou plusieurs thiolates de métal précieux, un ou plusieurs liants organiques polymères, en outre des solvants pour le ou les thiolates de métal précieux et le ou les liants, et, si nécessaire des adjuvants pour ajuster les propriétés de transformation de la préparation et les propriétés optiques et d'usage du décor contenant du métal précieux à produire avec elle,
caractérisée en ce qu'
elle contient un dithiolate de mono(métal précieux) selon la revendication 1 à 5 comme thiolate de métal précieux.

9. Préparation selon la revendication 8,
caractérisée en ce qu'
elle contient comme solvant un solvant organique polaire, notamment de la série des glycols C₂ à C₄, des oligoglycols C₂ à C₄, des monoéthers C₁ à C₄ des glycols cités et des oligoglycols, des acides hydroxycarboxyliques C₂ à C₃ et leurs esters alkyliques, et de l'eau.

10. Préparation de métal précieux selon la revendication 8,
caractérisée en ce qu'
elle contient comme solvant de l'eau et moins de 2 % en poids , par rapport à la préparation des solvants organiques, le liant est hydrosoluble, le dithiolate de mono-métal présente au moins un groupe fonctionnel pouvant former un sel et se présente sous la forme d'un sel hydrosoluble et en plus un tensioactif est présent en quantité efficace.

11. Préparation selon l'une des revendications 8 à 10,
caractérisée en ce qu'
on choisit le polymère organique dans la série des homo- ou copolymères de polyvinylpyrrolidone, des homo- ou copolymères de poly(acide acrylique) des homo- ou copolymères de poly(acide méthacrylique) et des éthers de cellulose hydrosolubles ainsi que des mélanges desdits liants.

12. Préparation selon la revendication 10 ou 11,
caractérisée en ce qu'
elle contient un sel hydrosoluble d'un dithiolate de mono(métal précieux) présentant un ou plusieurs groupes carboxyles et d'une amine primaire, secondaire ou tertiaire et d'une base N-hétérocyclique.

13. Préparation selon l'une des revendications 10 à 12,
caractérisée en ce que
le dithiolate de mono(métal précieux) est sous la forme d'un sel de trialkylamine C₁ à C₃ ou de mono- di- ou triéthanolamine et la préparation contient-en plus les adjuvants habituels de réglage des propriétés optiques et d'usage du décor à préparer et/ou des propriétés de transformation de la préparation.

14. Préparation selon l'une des revendications 8 à 13,
caractérisée en ce qu'
elle contient un sel hydrosoluble de l'acide mono-or (I)-dimercaptosuccinique.

15. Préparation selon l'une des revendications 10 à 14,
caractérisée en ce qu'
elle contient comme tensioactif 0,1 à 2,0 % en poids de préférence 0,2 à 1,0 % en poids d'un polyoxyéthylènepolydiméthylsiloxane.

16. Préparation selon l'une des revendications 10 à 15,
caractérisée en ce qu'
elle contient comme tensioactif un alkylbenzènesulfonate de 8 à 14 atomes de C dans le groupe alkyle, sous la forme d'un sel d'une amine en une quantité de 0,1 à 2 % en poids, de préférence 0,2 à 1 % en poids.

17. Décalcomanie pour produire des décors contenant des métaux précieux sur des supports à cuire, notamment du verre, de la porcelaine et de la céramique, contenant dans une couche décor, un ou plusieurs thiolates de métal précieux, un ou plusieurs liants organiques polymères et si nécessaire des adjuvants pour préparer la décalcomanie et pour modifier les propriétés optiques et d'usage du décor de métal précieux à produire,
caractérisée en ce qu'
un dithiolate de mono(métal précieux) selon l'une des revendications 1 à 5 est présent en répartition homogène comme thiolate de métal précieux.

18. Procédé pour décorer des substrats à cuire notamment du verre, de la porcelaine ou de la céramique par dépôt d'une préparation de métal précieux ou d'une décalcomanie comprenant à l'état sec une telle préparation à l'état séché sur le substrat et cuisson entre 400 et 900°C,
caractérisé en ce qu'
on dépose une préparation comprenant un métal précieux selon l'une des revendications 8 à 16 ou une décalcomanie selon la revendication 17.
